# EUROPEAN PATENT APPLICATION

(11) **EP 1 995 324 A1**
(43) Date of publication of application: **26.11.2008**
(21) Application number: 07010114.2
(22) Date of filing: 22.05.2007
(51) Int. Cl.: C12P 21/02, C07K 1/13

(54) **Cell-free protein synthesis of deuterated proteins for structural and functional studies by NMR spectroscopy**

(71) Applicant: ETH Zurich, 8092 Zürich (CH)
(72) Inventor: Etezady-Esfarjani, Touaj, 8053 Zurich (CH); Wüthrich, Kurt, 8304 Wallisellen (CH)

(57) **Abstract**

A method for cell-free synthesis of at least partially deuterated proteins, peptides or assemblies thereof, in particular for NMR studies, is described. The method comprises at least the following steps: a) preparation of a cell extract under essentially non-deuterated conditions; b) exchanging D₂O into the cell extract in a buffer exchange process to form a deuterated cell extract; c) cell-free synthesis using at least the deuterated cell extract, expressed and purified polymerase, at least partially deuterated amino acids and a plasimd or a linear template DNA encoding said protein or peptide; d) isolation and purification of the protein or peptide. Furthermore an extract for use in such a cell-free synthesis and a kit for use in such a cell-free protein synthesis is described

## Description

### TECHNICAL FIELD

The invention pertains to improved methods for cell-free protein synthesis e.g. of large molecular assemblies for NMR studies.

### BACKGROUND OF THE INVENTION

For many techniques used in the context of structural nuclear magnetic resonance spectroscopy (NMR) isotope labelled samples are not only useful but sometimes even indispensable.

As described for example in EP-A-1655375 or EP-A-1666603, cell-free protein synthesis can be of interest for the preparation of protein samples with non-uniform isotope labelling for NMR spectroscopy. In particular, it provides for efficient incorporation of selectively labelled amino acids into polypeptide chains in situations where in vivo protein expression results in isotope scrambling or isotope dilutions (see for example Kigawa, T., Muto, Y., and Yokoyama, S. Cell-free synthesis and amino acid-selective stable isotope labelling of proteins for NMR analysis. J. Biomol. NMR 6, 129-134 (1995) or Ozawa, K. et al, Optimization of an Escherichia coli system for cell-free synthesis of selectively 15N-labelled proteins for rapid analysis by NMR spectroscopy. Eur. J. Biochem. 271, 4084-4093 (2004)). This includes that cytotoxic proteins, such as proteases or apoptosis-related proteins, have been produced using a cell-free protein synthesis approach. Transcription-translation-coupled systems based on E. coli or wheat germ cell extracts in continuous exchange reaction chambers are widely used, with yields of several milligrams of protein per millilitre of reaction mixture. These fundamental procedures are increasingly being supplemented by eukaryotic cell-free systems generated from insect cells or hybridoma cell-lines, which allow further studies of post- and co-translational events such as N-myristoylation.

Most of the proteins produced so far with the aforementioned cell-free expression protocols are small monomeric structures, although in vitro expression with stereo-array isotope labelling (SAIL, see e.g. Kainosho, M. et al. Optimal isotope labelling for NMR protein structure determinations. Nature 440, 52-57 (2006) or Sugimori, N. et al. 1H, 13C and 15N backbone assignment of a 32 kDa hypothetical protein from Arabidopsis thaliana J. Biomol. NMR 30, 357-358 (2004)) has recently generated interest in cell-free production of larger proteins. For polypeptides in structure sizes above 40 kDa, it was documented that high levels of deuteration are indispensable for solution NMR studies. With in vivo expression in D₂O -based medium, perdeuterated proteins are, however, usually obtained only after lengthy "training" of the cells, albeit typically with significantly lower expression yields than in H₂O -based growth media.

### SUMMARY OF THE INVENTION

High deuteration levels required for solution NMR studies of large proteins, or small proteins incorporated in large supramolecular structures cannot be achieved with conventional protocols for cell-free protein synthesis, which typically yield low, non-uniform deuteration levels. Therefore, it is of keen interest to explore the use of cell-free synthesis also for the production of perdeuterated proteins. This document now introduces a new (E. coli) cell-extract, called D-S30, which enables efficient production of proteins with high deuteration levels for all non-labile hydrogen atom positions. The 800 kDa chaperonine GroEL synthesized with the D-S30 cell-free system had a uniform deuteration level of about 95%, and it assembled into its biologically active oligomeric form. The use of the D-S30 cell extract with the protocol described in this paper also enables production of proteins with selectively isotope-labelled amino acid residues on a perdeuterated background. In this document, a protocol is described that achieves this goal, as demonstrated with the synthesis of the 800 kDa E. coli chaperonine GroEL, which is an oligomeric protein with 14 identical subunits.

Specifically, a method for cell-free synthesis of at least partially deuterated proteins, peptides or assemblies thereof, in particular for NMR studies, is proposed, comprising at least the following steps, eventually including further subsequent or intermittent steps:
a) preparation of a cell extract under essentially non-deuterated conditions (preparation of the so-called S30 extract);
b) exchanging D₂O into the cell extract in a buffer exchange process to form a deuterated cell extract (preparation of the so-called D-S30 extract);
c) cell-free synthesis using at least the deuterated cell extract (D-S30), expressed and purified polymerase, at least partially deuterated amino acids and a plasimd or a linear template DNA encoding said desired protein or peptide;
d) isolation and purification of the protein or peptide, wherein this last step is adapted to the specific protein or peptide.

As will be shown in the following, it was unexpectedly found that conventional methods for producing deuterated proteins fail or are economically not viable.

Specifically, when e.g. using lyophilization techniques or gel filtration techniques, the activity in the cell-free protein synthesis goes down dramatically increasing raw material costs significantly and decreasing product quality. When using dialysis methods excessive amounts of D₂O required making this process difficult and costly. Furthermore it was noted that one major problem in the context of using cell-free synthesis for the production of deuterated proteins is the still unexplained problem of substitution or exchange of protons at certain positions of the amino acids used. Specifically, even if fully deuterated amino acids are used in the cell-free synthesis, during the actual cell-free synthesis at certain amino acid positions, namely at mainly the alpha and/or beta positions deuterons are exchange to protons for reasons still unclear. Unexpectedly this problem of exchange or replacement can be avoided in the proposed process.

One problem that one can face in the context of such cell-free synthesis protocols is the solubility of cell-free synthesized proteins. This means that indeed many protein can in principle be synthesized but their final solubility is rather low leading to efficiency problems. In order to overcome this, one can also prepare the above cell-extract from cells that have already produced (or better said expressed) helper molecules such as chaperones (e.g. GroEL is also a chaperone) or other molecules that enhance the folding properties of the newly synthesized protein. So when talking about a cell extract this also includes cell-extracts obtained from cells that have been manipulated to e.g. overproduce certain biological macromolecules.

Furthermore also the use of further additives (such as lipids, detergents, salts, ions, or other small or large molecules) to the cell extract prior to starting the cell-free reaction is possible. Thereby it is e.g. possible to provide or design different screening strategies.

Most effectively the above effect can be realised if in step b) a filter device is used with a specific molecular weight cut off in the range of 5000-25,000 Da.

It was specifically found that below 5000 Da and above 25000 Da the activity or productivity in the cell-free synthesis step goes down almost completely and additionally enhanced replacement is observed.

Even better results can be achieved if the filter device has a molecular weight cut off in the range of 8000-15,000 Da, preferably in the range of 8000-12,000 Da.

A preferable filter which can be used in this context is the Amicon-Ultra centrifugation filter with a molecular weight cut off (MWCO) of 10'000 Da. Correspondingly therefore, it is preferred to use a centrifugal filter device is used.

According to a further preferred embodiment of the present method the buffer exchange is carried out at least three times, preferably at least five times, even more preferably between 5 and 15 times, e.g. six times.

It is furthermore preferred to for step b) the sample from step a) is separated into at least two fractions, preferably at least three fractions, and wherein in step b) these fractions are treated in parallel using corresponding number of filter devices. a highly efficient process can be realised if with a sample of 15 mL from step a) 3 fractions are made of 5 mL and these are subsequently exchanged with a D₂O for a solution six times using three of the above-mentioned filter devices. Typically step b) is carried out at a temperature in the range of 4°C. Generally, centrifugation with less than 5000 g is used, and since going down with centrifugation will lead to longer filtering times and correspondingly increased problems associated with long storage and degradation of activity of the extract, preferably one works in the range of 4000 to 4500 g for the centrifugation step .

Specifically good results can be achieved if the whole process is carried out under conditions which make sure that RNase activity is kept low if not fully prevented. This is for example possible if prior to the use of the filter device in step b) said filter device is treated to prevent/remove RNase activity, preferably by using Diethylpyrocarbonate (DEPC). Similarly it is possible to treat the D₂O used in steps c) and c) to prevent/remove RNase activity, preferably by using DEPC, even more preferably in combination with subsequent autoclaving of the D₂O in a sealed container, for example during one hour at approx. 120°C.

A further preferred embodiment of the present invention is characterised in that in step a) the cell extract is supplemented with purified ribosomes, wherein preferably these ribosomes are based on an E. coli cell culture grown in incomplete rich medium, preferably harvested at OD600 of less than one, even more preferably preferably at OD₆₀₀ in the range of 0.7, with subsequent purification based on ultra-centrifugation and e.g. sucrose cushion approach, and wherein preferably these purified ribosomes are added after cell breakage and/or before filtration. These supplemented purified ribosomes can make up for partial degradation/removal of ribosomal activity of the cell extract in the filtering step and is thus unexpectedly boosting the overall efficiency of the process specifically in the context of the making of D-S30.

According to one embodiment of the invention the amino acids used in step c) are deuterated in non-labile hydrogen-positions preferably in at least the alpha and/or the beta-position. Preferably (all) the amino acids are fully deuterated. It is possible to have groups of fully deuterated amino acids in combination with groups of not deuterated amino acids, it is possible to have only fully deuterated amino acids in combination with one single type of amino acid which is not deuterated at all or not fully deuterated, or with a limited number of types of amino acids which are not deuterated at all or not fully deuterated.

According to a further embodiment of the present invention, the cell-free synthesis in step c) is carried out in batch mode or in continuous mode, wherein in the latter case preferably the ratio of reaction volume and feeding volume is in the range of 5 : 1 to 15 : 1.

According to another embodiment of the present invention, all the substrates for the cell-free synthesis in step c) are prepared in D₂O, wherein preferably in order to remove residual H₂O prior to the preparation of the substrates in D₂O, the corresponding stable salts are produced and incubated before dissolution in D₂O.

Particularly good results can be achieved if for the preparation of the S30 in step a) the E.coli strain BL21 Star is used. However it is also possible to base the cell extract S30 on other cell basis, so it may also be a e.g. wheat-germ or insect extract.

Furthermore the present invention relates to an extract for use in a cell-free synthesis according as described hereinabove, made in a process comprising at least the following steps: a) preparation of a cell extract under essentially non-deuterated conditions; b) exchanging D₂O into the cell extract in a buffer exchange process to form a deuterated cell extract.

In addition to that, the present invention relates to a kit for use in a cell-free protein synthesis comprising such an extract. A possible cell-free kit based on an extract as given above, is possible allowing fast screening of several different conditions within a short period of time, e.g. by adding tailored additives (such as lipids, detergents, salts, ions, or other large or small molecules) to the cell extract prior to the start of the cell-free reaction. I

Last but not least the present invention relates to the use of the method as described above or an extract as described above or a kit as described above for the preparation of an NMR-sample. This preferably for screening of small molecules interacting with large proteins by means of dipolar cross-relaxation, e.g. in a so-called SOS-NMR-approach (see e.g. SOS-NMR: a saturation transfer NMR-based method for determining the structure of protein-ligand complexes, P.J. Hajduk et al. J. Am. Chem. Soc. 2004, 126, 2390-2398).

Further embodiments of the present invention are outlined in the dependent claims.

### SHORT DESCRIPTION OF THE FIGURES

In the accompanying drawings preferred embodiments of the invention are documented in which:

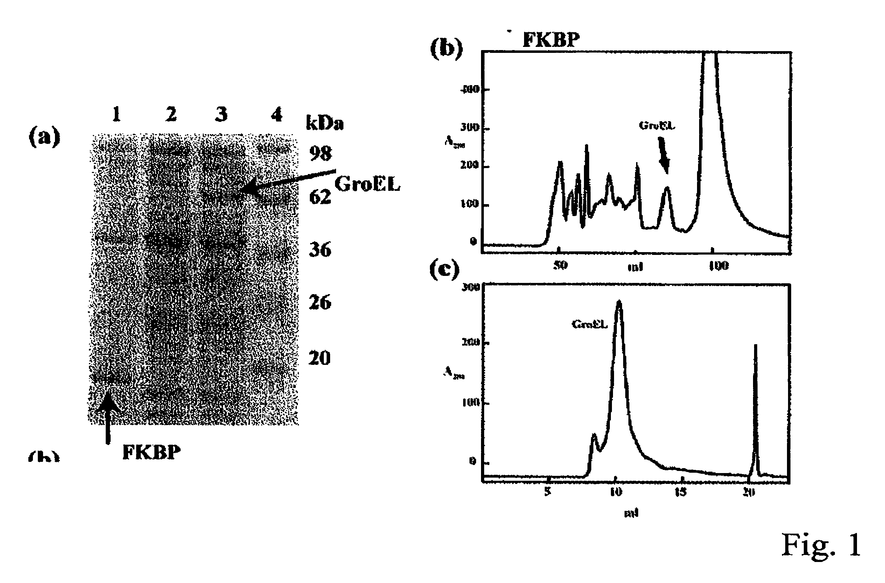

- Fig. 1: documents the cell free protein synthesis of GroEL and FKBP, wherein in (a) the translation efficiency of cell-free protein synthesis is monitored by SDS-Page, lanes: 1, FKBP synthesis using continuous-exchange cell-free protein synthesis (CECF), the yield is 1.5 mg/ml reaction mixture; 2, negative control; 3, GroEL synthesized by batch mode cell-free proteins synthesis, the yield is 300 mug/ml reaction mixture; 4, protein ladder with indication of molecular weights in kDa, wherein in (b) anion exchange chromatography for the purification of cell-free synthesized GroEL is shown, the protein elutes as a single peak, identified by an arrow and wherein in (c) analytical size exclusion chromatography of GroEL is shown, the elution volume of 10.4 ml corresponds to a particle size larger than 400 kDa (column specification);

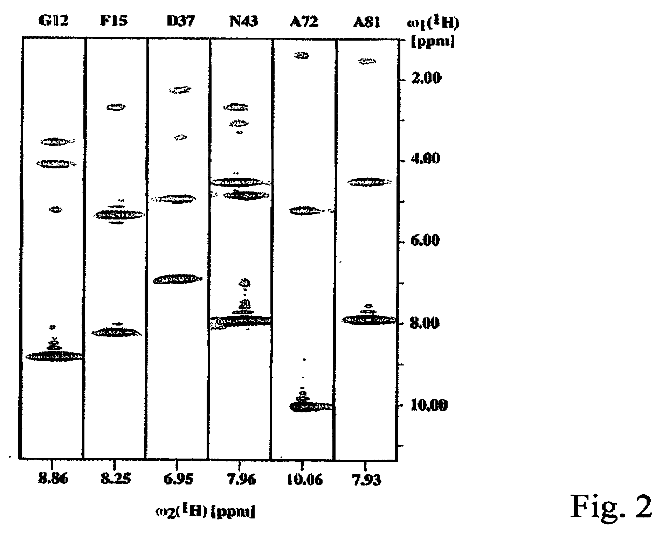

- Fig. 2: shows the back-protonation during the incorporation of perdeuterated amino acids by cell-free protein synthesis of FKBP in H₂O -based reaction mixture monitored with 3D 15N resolved [1H,1H]-TOCSY experiments (τₘ= 60 ms), wherein data are shown for 6 residues, where the H^{N}-N-H^{N} peaks, the H^{N}-N-H^{α} cross-peaks, and the H^{N}-N-H^{β} cross-peaks are visible, and wherein the NMR data were processed with the software PROSA29;

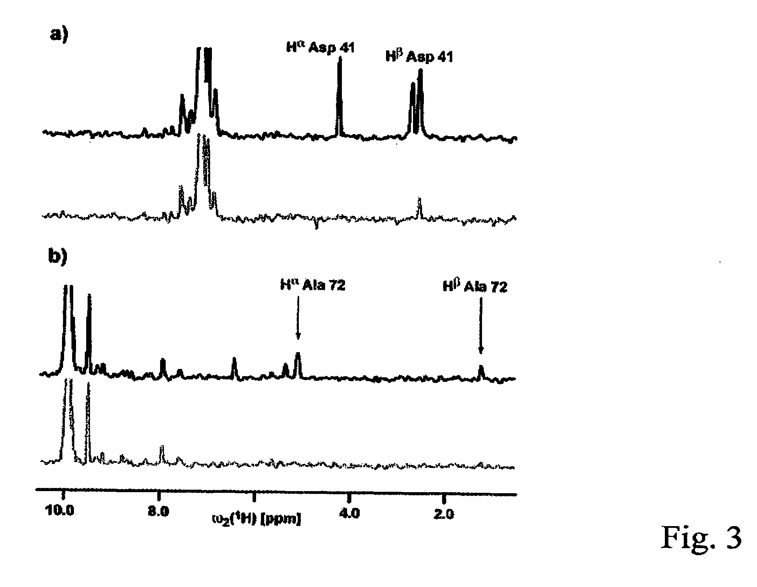

- Fig. 3: shows the amino acid-specific back-protonation monitored by comparison of 1D cross-sections taken from 2D ['H,'H]-NOESY experiments recorded with FKBP at 600 MHz with a mixing time of 100 ms, wherein in (a) Asp 41, (b) Ala 72 is shown, upper spectrum: sample prepared in H₂O -based S30 cell extract; lower spectrum: synthesis in D₂O -based D-S30 cell extract;

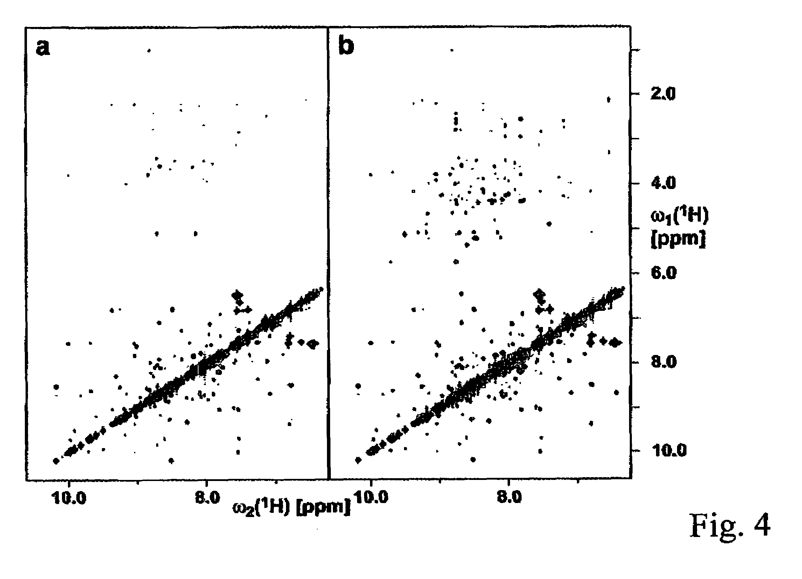

- Fig. 4: shows the back-protonation of perdeuterated amino acids during in vitro synthesis of FKBP monitored by comparison of the 600 MHz 2D [¹H, ¹H]-NOESY spectra of FKBP produced in vitro using either D₂O -based D-S30 cell extract (a) or H₂O -based S30-extract (b), wherein the protein concentrations were 800 µM, the measurements were carried out at 23°C with a mixing time of 100 ms, and the two spectra were recorded and processed identically; and

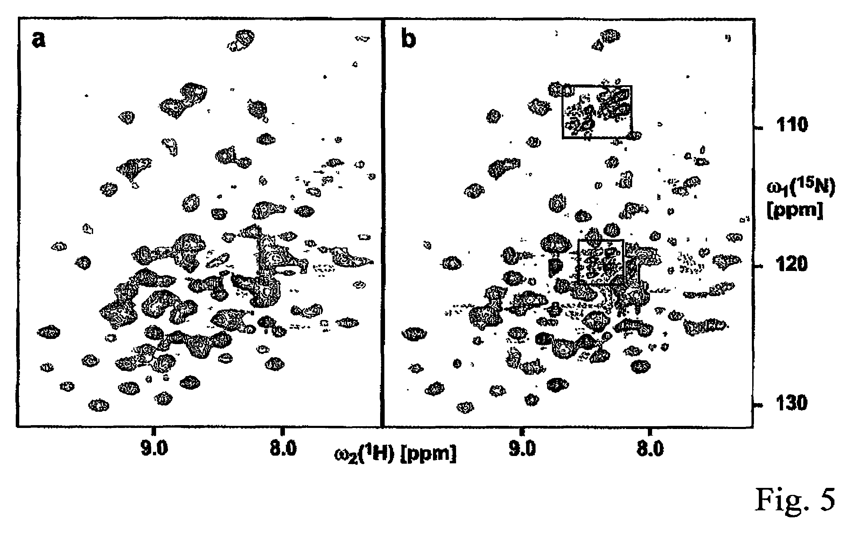

- Fig. 5: shows 2D [15N,1H]-CRIPT-TROSY NMR spectra of two different samples of uniformly ²H, ¹⁵N-labelled GroEL, wherein in (a) protein expressed in vivo in E. coli cells is shown and in (b) protein synthesized in vitro with D-S30 extract, wherein two spectral regions are highlighted in spectrum (b) by rectangles identifying local differences between the two spectra that are discussed in the text.

### DETAILED DESCRIPTION OF THE PREFERRED EMBODIMENTS

Referring to the drawings and the description hereinbelow, it shall be pointed out that these are for the purpose of illustrating the present preferred embodiments of the invention and not for the purpose of limiting the same, as defined in the appended claims.

### Cell-free expression of GroEL

Cell-free synthesis of GroEL was carried out using the batch mode synthesis approach, which is cost-effective for the expression of large amounts of protein due to the low amount of amino acids needed. This can be an important aspect, since about 30 mg of protein are required for an NMR sample with a concentration of 1 mM in monomers of GroEL. About 400 µg of GroEL are produced from 1 ml of reaction mixture (see Figure 1, a and b), of which about 55% are accessible in soluble form, as shown by analytical ultra-centrifugation. The formation of the native multimeric form was confirmed by analytical size exclusion chromatography (Figure 1c), which shows that the retention time and the elution profiles were almost identical to those of in vivo expressed GroEL.

### Production of perdeuterated proteins using H₂O -based S30 extract

As a first approach to cell-free production of perdeuterated proteins, the standard cell-free protein synthesis protocols based on Torizawa, T., Shimizu, M., Taoka, M., Miyano, H., and Kainosho, M. Efficient production of isotopically labeled proteins by cell-free synthesis: A practical protocol. J. Biomol. NMR 30, 311 (2004) as summarized in Table 1 was applied using a mixture of the 20 proteinogenic amino acids with U-98% deuteration as the amino acid source.

**Table 1: Composition of the reaction mixture and the feeding mixture in continuous-exchange cell-free proteins synthesis setup. In the batch setup, the reaction was carried out in the reaction mixture for 4-5 hours at 30°C and 450 rpm.**

| **Components** | **Reaction mix (1 ml)** | **Feeding mix (10 ml)** |
|---|---|---|
| S30-extract (OD₂₈₀, 200) | 330 µl | - |
| Plasmid DNA | 10 µg/ml | - |
| T7-RNA polymerase | 20 µl | - |
| | | |
| *E. coli* tRNA | 240 µg/ml | 66 µg/ml |
| 20 amino acids Mix | 1.5 mM (each) | 1.5 mM (each) |
| Creatine kinase | 83 µg/ml | |
| Phosphocreatine | 84 mM | 84 mM |
| ATP | 1.27 mM | 1.27 mM |
| CTP, GTP, UTP | 0.9 mM (each) | 0.9 mM (each) |
| cAMP | 0.67 mM | 0.67 mM |
| Folinic acid | 36 mM µg/ml | 36 µg/ml |
| DTT | 2.1 mM | 2.1 mM |
| Tris-acetate, pH 8.2 | 3 mM | 3 mM |
| Hepes-KOH pH 7.5 | 60 mM | 60 mM |
| NH4OAc | 29 mM | 29 mM |
| Magnesium acetate | 11.2 mM | 11.2 mM |
| Potassium acetate | 20 mM | 20 mM |
| Potassium glutamate | 210 mM | 210 mM |
| Polyethylenglycol 8000 | 4% (w/v) | 4% (w/v) |
| Sodium Azide | 0.025% (w/v) | 0.025% (w/v) |

The relative expression level with deuterated amino acids was about 95% of the yield in cell-free synthesis of GroEL using unlabeled amino acids. However, mass spectroscopy of the protein showed a 0.5% smaller mass than expected for the fully perdeuterated protein, which could be due to the incorporation of non-labelled amino acids present in the S30 extract. To eliminate this source of unlabeled amino acids, the protocol used for the preparation of S30 extract was supplemented with an additional size exclusion chromatography step. Absence of residual amino acids in the resulting S30 extract was confirmed by the observation that immune-blotting assays for protein synthesis in reaction mixtures without amino acid supplementation did not show any detectable bands of newly synthesized protein. Nonetheless, the deuteration level of the protein expressed with perdeuterated amino acids did not increase significantly. 1D ¹H-NMR spectra of a sample of 31 mg of deuterated GroEL prepared in a 100 ml reaction volume using the batch mode indicated a high degree of back-protonation at the H^{α} positions, and to a lesser extent also at the H^{β} positions. In contrast, the high-field region of the ¹H NMR spectrum showed a deuteration level of about 95% for the methyl groups of the protein.

For a more detailed investigation of the α- and β-protonation levels resulting from cell-free production with perdeuterated amino acids we used the 110-residue FK506 binding protein (FKBP), for which the chemical shift assignments are known.

A reaction volume of 4 ml in a CECF setup produced 6.3 mg of FKBP, resulting in a 1.1 mM NMR sample. NMR experiments confirmed the observation of partial back-protonation at the α- and β- positions (see Figure 2), confirming that it is the exchange of H*^{α}* and H^{β} with bulk water during the polypeptide synthesis in the cell-free system that prevents perdeuteration of the protein.

Cell-free synthesis of several other soluble and membrane proteins in both batch and CECF setups yielded similar results to those with FKBP.

Semi-quantitive analysis of 2D [¹H, ¹H]-NOESY and 3D ¹⁵N-resolved [¹H, ¹H]-TOCSY spectra recorded with FKBP further revealed that the back-exchange is amino acid-specific, and that the 20 amino acids can be classified in four groups with different relative back-protonation levels (Table 2).

**Table 2: Amino acid-dependent back-protonation at the α- and β-positions in the course of cell-free protein synthesis. The classification is based on semiquantitative analysis of 3D ¹⁵N-resolved [¹H, ¹H]-TOCSY and 2D [¹H, ¹H]-NOESY spectra.**

| **Amino acids** | **Relative degree of α- and β-back-protonation** |
|---|---|
| Asp, Asn, Gln, Glu, Gly | very high |
| Ala, Cys, Phe, Ser, Trp, Tyr | high |
| Arg, Ile, Leu, Lys, Val | medium |
| Thr, His, Met | low |

Gly, Asp, Asn, Glu and Gln show high back-protonation at the a-position, Ser, Cys, Ala, Phe, Trp and Tyr are a second group with intermediate back-protonation at the α-position, Val, Ile, Leu Lys and Arg have a low degree of back-protonation, and Thr, His and Met show almost no back-protonation. Asp, Asn, Gln and Glu have high back-protonation also at the (3-position (Figure 2), whereas Ala, Phe and Tyr showed less extensive (3 back-protonation (see Figure 2).

### Preparation of deuterated S30 extract for cell-free synthesis (D-S30)

In order to achieve higher deuteration levels at the a- and [3-positions by cell-free protein synthesis with [µ-98% ²H]-amino acids, we successively reduced possible proton sources in the extract preparation, and monitored the results by MS and NMR spectroscopy (see Figure 3). In the cell-free synthesis with the batch setup, 33% of the reaction volume is comprised of S30 extract, which was thus a prime target for modifications aimed at reduced back-protonation. In principle, deuterated S30 extract could be obtained from cells grown in a D₂O -based medium. However, since the harsh growth conditions in D₂O reduce the overall fitness of bacterial cells, we would expect the activity of such a cell-extract to be reduced. Further, since about 18 liters of D₂O would be needed for obtaining 15 ml of cell-extract, this approach would not be cost-effective. We therefore chose the alternative of the exchanging of D₂O into S30 extract that had been prepared on a H₂O -basis, either by filtration, dialysis or lyophilization. In all assays, the activity of the resulting D-S30 extract was compared to the activity of standard S30 extract. High activity with reasonable buffer exchange costs was achieved by filtration. In a series of preparations with different filtration devices covering molecular weight cutoffs (MWCO) between 3'000 and 50'000 Da, H₂O was replaced by D₂O.

The resulting extracts were compared with each other by their protein synthesis efficiency as monitored by Western blotting. Optimal synthesis efficiency was obtained with a MWCO of 10'000 Da. MWCO values below 10'000 resulted in the formation of larger amounts of precipitates, which could not be resuspended, and MWCO values above 10'000 resulted in reduced extract activity. The translation efficiency of the D-S3 0 extract thus obtained was about 65% of that of the conventional S30 extract. For the transformation of S30 to D-S30 extract we used six filtration cycles with a dilution factor of 2, resulting in a D₂O level above 98%. A higher number of filtration cycles resulted in reduced translation efficiency of the D-S30 extract In spite of the residual H₂O content in all other components of the cell-free reaction setup, the final H₂O concentration in D-S30 was less than 5%. Different results were achieved with a conventional dialysis approach, resulting in 60-70 % of the activity of S30 extract, so much lower activity, and due to the large volume of D₂O needed for complete buffer exchange, this approach is economically not viable. In contrast, lyophilization turned out to be inadequate, since only a partial resuspension of the lyophilized extract was achieved, with nearly zero remaining activity of the cell-extract.

The D-S30 extract was supplemented with purified ribosomes prior to the filtration step, which further increased the overall efficiency of the synthesis reaction up to about 85% of the efficiency of the conventional S30 extract without ribosome supplementation (see Material and Methods below).

### Comparison of deuteration levels obtained with S30 and D-S30 extract

The figures 3 and 4 show comparisons of 2D [¹H, ¹H]-NOESY NMR data of FKBP produced in vitro using either the conventional S30-extract or the new D-S30 extract. All the spectra were recorded on a 600 MHz spectrometer with a mixing time of 100 ms, and processed identically. The protein concentration was adjusted to 800 µM as determined by UV absorption spectra. The high level of back-protonation in the protein prepared with conventional S30-extract is manifested by the appearance of numerous H^{N}-H^{α} and H^{N}-H^{β} cross-peaks (Figures 3 and 4b). Most of the H^{N}-H^{α} cross-peaks in Figure 4b originate from Asp (see Figure 3a), Asn, Glu and Gln residues, which also show a high tendency for back-protonation in the β-position. In addition, the back-protonation of the Ala methyls is rather striking (see Figure 3b). The reduced back-protonation in the FKBP preparation from the D-S30 extract is reflected by the dramatically reduced peak intensities in the cross sections of Figure 3, and by the low population of and H^{N}-H^{β} cross peaks in Figure 4a. There is some residual back-protonation at 13-positions for the amino acids Asp (Figure 3a) and Asn, but it is much lower than with the conventional S30 extract.

### GroEL synthesis with D-S30 extract

The translation efficiency of the new D-S30 extract for the synthesis of GroEL with unlabelled or with perdeuterated amino acids was similar, and it was also comparable to the reaction yield with FKBP. The elution profiles were similar to those shown in Figure 1 for protonated GroEL. A MS analysis of GroEL from deuterated amino acids synthesized in D-S30 extract showed a deuteration level of about 95%. 2D [¹⁵N,¹H]-CRIPT-TROSY spectra of GroEL synthesized with the D-S30 extract and of GroEL prepared by in vivo protein expression show high similarity of the two samples (see Figure 5), indicating that proper assembly of cell-free synthesized GroEL monomers into the biologically active 14-mer was achieved. A potentially interesting detail is that the peak multiplets contained in the red boxes (Figure 5b) have higher intensity than the signals at the same chemical shifts in the in vivo produced GroEL. From their fine structure in the 2D [¹⁵N,¹H]-CRIPT-TROSY spectrum, these signals must arise from a flexibly disordered polypeptide segment. A probable candidate is the 25-residue C-terminal tail of GroEL, which contains four GGM motifs. The indication thus is that this tail is present in the protein from in vitro synthesis, whereas it seems to be largely degraded in the in vivo expressed GroEL.

### Discussion

From the NMR viewpoint, back-protonation of perdeuterated amino acids at the α- and β-positions during in vitro protein synthesis in H₂O-based cell extract (Figure 2) inevitably results in the deterioration of measurements with large structures, in particular when using TROSY-based experiments. This document now describes an economically viable protocol for cell-free synthesis of perdeuterated proteins. It is based on the preparation of the deuterated D-S30 cell extract, which yields 95% deuteration for non-labile proton positions with similar expression levels as the H₂O-based S30 extract.

In addition to the production of perdeuterated proteins, the new protocol can be adapted for more sophisticated labelling schemes, which may not readily be accessible by other techniques. For example, selective incorporation of individual ¹H,¹⁵N,¹³C-labeled amino acid types into proteins on a uniform ²H,¹⁴N,¹²C-labelling background could open new perspectives for studies of active site screening of enzymes. The approach used here with E. coil cell extract should in principle also be adaptable to other cell-free systems, for example, using wheat germ or insect cell extract for the production of eukaryotic proteins.

For practical reasons it is of interest to further investigate the mechanisms leading to back-protonation of deuterated amino acids during cell-free synthesis in H₂O-based cell extract (Figure 2). The observations that strong back-protonation at the α-position is observed only for some of the amino acids (Table 2) and that back-protonation at the β-position is even more selective, indicates that back-protonation could be due to amino acid type-specific enzymatic reactions in the cell extract. We have so far considered racemase activities as a likely cause, and therefore focused on screening for racemase activities in the cell-extract. Thereby we made use of the fact that D-glutamate and D-aspartate are important components of peptidogylcan synthesis and are therefore essential for bacterial cell-wall biosynthesis. D-Glu and D-Asp are synthesized from L-Glu and L-Asp, respectively, by specific glutamate- and aspartate racemases. To make use of the reverse reactions, we used D-Glu and D-Asp instead of their L-counterparts in the cell-free protein synthesis setup, and the amount of protein produced with this variant amino acid mixture was monitored by an immunoassay using anti-His antibodies. The results of these experiments did not indicate any significant protein production, and we therefore conclude that other factors than racemase activities in the cell-extract must have a role in the observed back-protonation.

Although a generalization based on work with a single system would be premature, the present results with GroEL indicate that proper assembly of oligomeric proteins can be achieved under cell-free conditions. It thus seems worthwhile to further explore the use of cell-free protein synthesis for the production of multi-subunit proteins.

### Experiments:

### Materials and Methods

### Preparation of S30 and D-S30 extract

For the preparation of S30 extract, the E. colt strain BL21 Star (DE3; Invitrogen) was used, since the cell extract from this strain showed a better performance than the S30 extract obtained from regular BL21 (DE3) or BL21 (DE3) RIL strains. The production of S30 extract was based on previously reported protocols (Torizawa, T., Shimizu, M., Taoka, M., Miyano, H., and Kainosho, M. Efficient production of isotopically labeled proteins by cell-free synthesis: A practical protocol. J. Biomol. NMR 30, 311 (2004); Kigawa, T. et al. Preparation of Escherichia coli cell extract for highly productive cell-free protein expression. J. Struct. Funct. Genomics 5, 63-68 (2004); Kigawa, T. et al. Cell-free production and stable-isotope labeling of milligram quantities of proteins. FEBS Lett. 442, 15-19 (1999)), with modifications that increased the efficiency of protein synthesis.

Routinely, 9 litres of incomplete rich medium containing yeast extract (10 g/l), glucose (1% w/v), KH₂PO₄ (40 mM) and K₂HPO₄ (165 mM) were inoculated with 40 ml of overnight culture. The cells were harvested at an early phase of exponential growth (OD₆₀₀ of 0.7), and were then washed three times with 400 ml of ice-cold S30 buffer containing 7 mM ss-mercaptethanol, 1 mM dithiothreitol, 14 mM magnesium acetate, 60 mM potassium acetate and 10 mM Tris-acetate at pH 8.2.

Cell-breakage was carried out with a single passage of cell suspension (0.8 g/ml) in S30 buffer through a French-Press at 15'000 psi, followed by two rounds of centrifugation of the cell-lysate at 30'000g at 4 °C.

In order to increase the translation efficiency, the cell extract thus obtained was supplemented with purified ribosomes that had been isolated from 9 litres of E. coli cell culture grown in incomplete rich medium also harvested at OD₆₀₀ of 0.7. Ribosome purification was based on ultra-centrifugation and a sucrose cushion approach. For best efficiency of these added ribosomes in the cell-extract it was found to be optimal to add the additional ribosomes just before the step which is required for the removal of endogenous mRNA (see next paragraph).

For removal of the endogenous mRNAs, 1 ml of freshly obtained cell-lysate was incubated for 60 minutes at 25 °C with 155 µl of a solution containing 13 mM ATP, 40 RM of each of the 20 proteinogenic amino acids, 4 mM DTT, 9 mM magnesium acetate, 85 mM phosphoenolpyruvate, 20 units of pyruvate kinase and 290 mM Tris-acetate at pH 8.2. After incubation, the extract was dialyzed three times against 11 of S30 buffer at 4 °C before an additional overnight dialysis against 21 of S30 buffer at 4 °C using a Spectra/pore membrane with a molecular weight cut off (MWCO) of 3'000 Da.

These extensive dialysis steps ensured complete removal of endogenous amino acids from the cell extract. Using a dialysis step against polyethylene glycol 8000 (PEG-8000)/S30 buffer (50% w/w), the final extract volume was reduced to 15 ml, and after additional centrifugation for 10 min at 4'000g and at 4 °C, the S30 extract was stored at -80 °C until further use.

For the preparation of D-S30 extract, the S30 extract was divided into three 5 ml fractions and then treated in parallel using three centrifugal filter devices (MWCO 10'000 Da), and buffer exchange against an equal volume of deuterated S30 buffer was repeated six times.

### Expression and purification of T7 RNA polymerase

Expression and purification of T7 RNA polymerase was based on a simplified method using the pT7-911 vector, which is derived from pQE-8 (Qiagen) encoding the T7 RNA polymerase (RNAP) gene with a poly-His₆ purification tag at the N-terminus. RNAP was expressed in E. coli cells BL21 (DE3) (Stratagene) using a standard procedure with induction of protein expression by the addition of 1 mM isopropyl (3-D-thiogalactopyranoside (IPTG). Cell breakage was carried out by several passages through a French-Press at 20'000 psi, and protein purification was carried out by affinity chromatography using a Ni-NTA-agarose resin column followed by size exclusion chromatography.

### Cell-free protein synthesis

The analytical cell-free protein syntheses of FKBP and GroEL were carried out in batch mode. Depending on the overall reaction yield and the isotope consumption, the largescale reaction was carried out either in the batch mode or by continuous exchange cell-free proteins synthesis. The nutrient compositions for batch and CECF mode are summarized in Table 1 as given above. The ratio of reaction volume and feeding volume in the CECF setup was 10:1. For the production of deuterated proteins, all the substrates for the cell-free protein synthesis reaction were prepared in D₂O.

In order to remove residual water prior to the preparation of the substrates in D₂O, the stable salts (Table 1) were incubated at 200 °C for 2 hours before they were dissolved in D₂O. The reaction mixture was incubated at 30 °C and 450 rpm either for 5 hours (batch mode), or for 17 hours (CECF mode), using a tabletop thermomixer (Eppendorff).

### Protein purification

Purification of FKBP was based on affinity chromatography using a Ni-NTA-agarose resin column and a linear gradient of imidazole. After the cell-free reaction, the reaction and feeding volumes were collected and centrifuged for 10 min at 15'000 g. The supernatant was loaded on the Ni-NTA-agarose resin column equilibrated with buffer A (30 mM imidazole, 500 mM NaCl, 20 mM sodium phosphate, pH 7.2) and the column was washed with three volumes of this buffer. FKBP was eluted from the column with a linear gradient of imidazole (0-500 mM, 5 column volumes) in buffer A, together with RNAP. An additional size exclusion chromatography was carried out to separate RNAP from FKBP, using a Superdex 75 prep grade (GE Healthcare) column equilibrated in NMR buffer (20 mM sodium phosphate, pH 6.2). The fractions containing FKBP were pooled and concentrated to a final volume of 500 µl. Typically, protein concentrations of about 500 µM (5 mg of protein) were obtained from a 4 ml continuous exchange cell-free protein synthesis assay.

The purification of in vivo deuterated GroEL was based on previously reported isolation methods (Xu, Z., Horwich, A.L., and Sigler, P.B. The crystal structure of the asymmetric GroEL-GroES-(ADP)7 chaperonin complex. Nature 388, 741-750 (1997)). Cells carrying a plasmid for GroEL overexpression were grown under ampicillin control in D₂O-based minimal medium, and overexpression was induced with IPTG. 2-3 g of harvested cells were resuspended in 30 ml of extraction buffer (100 mM TrisCl, 1mM DTT, 0.1 mg/ml DNase I, pH 8.0) and lysed by sonication followed by centrifugation for 30 min at 30'000 g and 4 °C. At this point, only a single step of ammonium sulphate precipitation (40 % w/v) was carried out, and after another centrifugation step the supernatant was applied to a HighPrep 26/10 desalting column (GE Healthcare). This was followed by anion exchange chromatography using a DEAE-sepharose fast flow column equilibrated in Buffer B (50 mM TrisCl, 2 mM DTT, pH 7.2). Prior to elution, the column was washed with three column volumes of buffer B, and GroEL was eluted using a linear gradient of NaCl (0-500 mM, 5 column volumes) in buffer B. The fractions containing GroEL were concentrated and loaded on a Superdex 200 prep grade column equilibrated with NMR buffer (25 mM potassium phosphate, 20 mM KCI, pH 6.2).

For in vitro synthesized GroEL, the ammonium sulphate precipitation step was omitted. Despite similar elution profiles of in vivo and in vitro produced GroEL from the gel filtration column, insoluble aggregates were immediately formed upon addition of 40% w/v ammonium sulphate to the reaction mixture. Therefore, after the initial DEAE-sepharose fast flow column and the following desalting step, an additional anion exchange chromatography step was carried out using a high resolution Resource Q (GE Healthcare) column equilibrated in buffer B (50 mM TrisCl, 2 mM DTT, pH 7.2). GroEL was eluted from the column using a linear gradient of NaCl (0-500 mM, 10 column volumes). Finally, fractions containing

GroEL were concentrated and subjected to size exclusion chromatography, as described for in vivo expressed GroEL.

### Collection of NMR data

All NMR experiments with FKBP were recorded at 25 °C on a Bruker DRX600 spectrometer equipped with a standard triple resonance probehead. The protein concentration was adjusted to 0.8 mM for each sample. For the 3D ¹⁵N-resolved. [¹H,¹H]-TOCSY experiment with a mixing time of τₘ = 60 ms, 8 transients were added, resulting in a total measurement time of 39 h. The interscan delay was 1 s; 1024 complex points were recorded with an acquisition time of 131 ms, and prior to Fourier transformation the FID was multiplied with a 75°-shifted sine bell and zero-filled to 2048 complex points. In the ω₁(¹H)-dimension, 95 complex points were measured, with a maximal evolution time of 12 ms, and the data was multiplied with a 75°-shifted sine bell and zero-filled to 256 complex points before Fourier transformation. In the ω₂(¹⁵N)-dimension, 42 complex points were measured, with a maximal evolution time of 21 ms, and the data was multiplied with a 75°-shifted sine bell and zero-filled to 128 complex points before Fourier transformation. For the 2D [¹H, ¹H]-NOESY experiments with a mixing time of τₘ = 100 ms, 128 transients were added, resulting in a total measurement time of 20 h. The interscan delay was 1 s; 1024 complex points were recorded with an acquisition time of 128 ms, and prior to Fourier transformation the FID was multiplied with a 75°-shifted sine bell and zero-ruled to 2048 complex points. In the ω₁(¹H)-dimension, 256 complex points were measured, with a maximal evolution time of 32 ms, and the data was multiplied with a cosine function and zero-filled to 512 complex points before Fourier transformation.

For GroEL produced by cell-free expression, a 2D [¹⁵N,¹H]-CRIPT-TROSY spectrum was recorded at 35 °C on a Bruker Avance900 spectrometer equipped with a standard triple resonance probehead. The protein concentration was 0.7 mM in monomers, and the transfer time was T = 1.4 ms. 4048 transients were added with an interscan delay of 300 ms, resulting in a total measurement time of 4 days. In the ω₂ (¹H)-dimension, 1024 complex points were recorded with an acquisition time of 81 ms. Prior to Fourier transformation the FID was multiplied with a cosine function and zero-filled to 2048 complex points. In the ω₂ (¹⁵N)-dimension, 100 complex points were measured, with a maximal evolution time of 20 ms, and prior to Fourier transformation the data was multiplied with a 20°-shifted sine bell function and zero-filled to 256 complex points.

For GroEL prepared in vivo, a 2D [¹⁵N,¹H]-CRIPT-TROSY experiment was recorded with the same conditions as for the cell-free preparation, except for the following: the experiment was recorded on a DRX750 spectrometer, the protein concentration was 1.9 mM in monomers, 512 transients were added, the acquisition time was 97 ms, in the ω₂ (¹⁵N)-dimension the maximal evolution time was 22 ms, and the total measurement time was 12 hours.

In all experiments, the baseline was corrected using the IFLAT method in the direct dimension, and with polynomials of 2^{nd} order in the indirect dimension(s).

## Claims

1. A method for cell-free synthesis of at least partially deuterated proteins, peptides or assemblies thereof, in particular for NMR studies, comprising at least the following steps:
a) preparation of a cell extract under essentially non-deuterated conditions;
b) exchanging D₂O into the cell extract in a buffer exchange process to form a deuterated cell extract;
c) cell-free synthesis using at least the deuterated cell extract, expressed and purified polymerase, at least partially deuterated amino acids and a plasimd or a linear template DNA encoding said protein or peptide;
d) isolation and purification of the protein or peptide.

2. Method according to claim 1, wherein in step b) a filter device is used with a molecular weight cut off in the range of 5000-25,000 Da.

3. Method according to claim 2, wherein the filter device has a molecular weight cut off in the range of 8000-15,000 Da, preferably in the range of 8000-12,000 Da.

4. Method according to any of claims 2 or 3, wherein a centrifugal filter device is used.

5. Method according to any of claims 2 to 4, wherein the buffer exchange is carried out at least three times, preferably at least five times, even more preferably between 5 and 15 times.

6. Method according to any of claims 2 to 5, wherein for step b) the sample from step a) is separated into at least two fractions, preferably at least three fractions, and wherein in step b) these fractions are treated in parallel using corresponding number of filter devices.

7. Method according to any of claims 2 to 6, wherein step b) is carried out at a temperature in the range of 4°C and using centrifugation with less than 5000 g, preferably in the range of 4000 to 4500 g.

8. Method according to any of claims 2 to 7, wherein prior to the use of the filter device in step b) said filter device is treated to prevent/remove RNase activity, preferably by using Diethylpyrocarbonate.

9. Method according to any of the preceding claims, wherein in step a) the cell extract is supplemented with purified ribosomes, wherein preferably these ribosomes are based on an E. coli cell culture grown in incomplete rich medium, preferably harvested at OD₆₀₀ of less than one, even more preferably at OD₆₀₀ in the range of 0.7, with subsequent purification based on ultra-centrifugation and sucrose cushion approach, and wherein preferably these purified ribosomes are added after cell breakage and/or before filtration and/or just before the removal of endogenous mRNA.

10. Method according to any of the preceding claims, wherein the amino acids used in step c) are deuterated in non-labile hydrogen-positions preferably in at least the alpha and/or the beta-position, wherein even more preferably the amino acids are fully deuterated.

11. Method according to any of the preceding claims, wherein selectively one or a group of the amino acids used in step c) is not deuterated while the other amino acids are fully deuterated.

12. Method according to any of the preceding claims, wherein all the amino acids used in step c) are fully deuterated.

13. Method according to any of the preceding claims, wherein the cell-free synthesis in step c) is carried out in batch mode or in continuous mode, wherein in the latter case preferably the ratio of reaction volume and feeding volume is in the range of 5 : 1 to 15 : 1.

14. Method according to any of the preceding claims, wherein all the substrates for the cell-free synthesis in step c) are prepared in D₂O, wherein preferably in order to remove residual H₂O prior to the preparation of the substrates in D₂O, the corresponding stable salts are produced and incubated before dissolution in D₂O.

15. Method according to any of the preceding claims, wherein the D₂O used in steps c) and c) is treated to prevent/remove RNase activity, preferably by using Diethylpyrocarbonate, even more preferably in combination with subsequent autoclaving of the D₂O in a sealed container.

16. Method according to any of the preceding claims, wherein in step a) an E.coli strain is used or an extract is provided as a wheat germ or insect extract, wherein preferably the E.coli strain BL21 Star is used.

17. An extract for use in a cell-free synthesis according to any of the preceding claims, made in a process comprising at least the following steps:
a) preparation of a cell extract under essentially non-deuterated conditions;
b) exchanging D₂O into the cell extract in a buffer exchange process to form a deuterated cell extract.

18. Kit for use in a cell-free protein synthesis comprising the extract according to claim 17.

19. Use of the method according to claims 1-16 for the preparation of an NMR-sample preferably for screening of small molecules interacting with large proteins by means of dipolar cross-relaxation.
